# DEMANDE DE BREVET EUROPEEN

(11) **EP 4 245 221 A1**
(43) Date de publication de la demande: **20.09.2023**
(21) Numéro de dépôt: 23161687.1
(22) Date de dépôt: 14.03.2023
(51) Int. Cl.: A61B 5/18, A61B 5/00, B64D 45/00

(54) **DISPOSITIF ÉLECTRONIQUE DE SURVEILLANCE D'UN ÉTAT NEUROPHYSIOLOGIQUE D'UN OPÉRATEUR DANS UN POSTE DE COMMANDE D'UN AÉRONEF, PROCÉDÉ DE SURVEILLANCE ET PROGRAMME D ORDINATEUR ASSOCIÉS**

(30) Priorité: 15.03.2022 FR 2202250
(71) Demandeur: THALES, 92400 Courbevoie (FR)
(72) Inventeur: BERTHELOT, Bastien, 31036 Toulouse (FR)
(74) Mandataire: Lavoix

(57) **Abrégé**

L'invention concerne un dispositif électronique de surveillance (22) d'un état neurophysiologique d'un opérateur (14) dans un poste de commande (16) d'un aéronef (12) comprenant :
- un module de réception configuré pour recevoir une donnée d'un capteur (24);
- un module de catégorisation configuré pour associer à l'opérateur (14) une catégorie à partir des données reçues ;
- un module de traitement configuré pour extraire de chaque donnée au moins un paramètre représentatif de l'état neurophysiologique de l'opérateur (14) ;
- un module de détection configuré pour appliquer un modèle issu d'une méthode d'apprentissage automatique pour déterminer en fonction des paramètres représentatifs si l'opérateur (14) est dans un état neurophysiologique nominal ou dans un état neurophysiologique altéré, le modèle étant choisi parmi une liste de modèles prédéterminés en fonction de la catégorie associée à l'opérateur.

## Description

La présente invention concerne un dispositif électronique de surveillance d'un état neurophysiologique d'un opérateur dans un aéronef.

L'invention concerne également un procédé de commande de surveillance d'un état neurophysiologique d'un opérateur dans un aéronef.

L'invention concerne également un programme d'ordinateur comportant des instructions logicielles qui, lorsqu'elles sont exécutées par un ordinateur, mettent en oeuvre un tel procédé.

L'aéronef est typiquement un avion, un hélicoptère, ou encore un drone. L'opérateur est par exemple le pilote de l'aéronef, le copilote ou un opérateur radar. Le poste de commande est notamment disposé dans l'aéronef ou est disposé à distance de l'aéronef dans le cas d'un drone par exemple.

La surveillance de l'état neurophysiologique d'un tel opérateur est indispensable pour la sécurité de l'aéronef afin de détecter tout état délétère : fatigue, charge mentale, incapacitation, stress, etc, qui pourrait entrainer une incapacité de l'opérateur ou une détérioration de sa capacité à réaliser les taches prévues dans des conditions opérationnelles de vol.

De manière conventionnelle, l'état neurophysiologique d'un opérateur est surveillé par les autres opérateurs de l'aéronef. Par exemple le co-pilote surveille l'état neurophysiologique du pilote, et inversement.

Pour compléter cette surveillance, il a été proposé une surveillance au moyen d'un ou de plusieurs capteurs disposés dans l'aéronef. Les capteurs mesurent une certaine donnée et l'état neurophysiologique de l'opérateur en est déduit.

Toutefois, une telle méthode ne donne pas entière satisfaction. En effet, cette méthode n'est pas assez robuste. En particulier, il est nécessaire que la méthode de surveillance soit robuste aux variabilités des situations rencontrées. En effet, la détection d'un état neurophysiologique altéré doit être robuste à la variabilité des caractéristiques physiologiques des différents opérateurs (âge, genre, etc.), mais aussi à la variabilité de l'environnement dans lequel se trouve l'opérateur (cockpit d'avion en turbulence, cockpit où la luminosité ambiante varie, etc.).

En outre, la méthode doit être robuste malgré le peu de données opérationnelles à disposition correspondant aux comportements recherchés. Dans le domaine militaire, ce problème est d'autant plus complexe que les données collectées et utiles à l'application d'intérêt ne sont pas nécessairement disponibles puisque souvent classifiées, et peu nombreuses en raison de la difficulté d'observation de situations critiques.

Pour répondre à cette exigence, des méthodes ont été proposés afin de surveiller uniquement un état mental de l'opérateur en particulier comme par exemple la fatigue ou la charge mentale, etc. La méthode est alors entraînée sur une quantité restreinte de données. Bien que la surveillance puisse être satisfaisante à l'entraînement de l'algorithme, la généralisation du modèle, c'est-à-dire son adaptation à un nouveau sujet inconnu, engendre souvent une forte baisse des performances. Une telle méthode est donc très spécifique aux données sur lesquelles l'entrainement est réalisé et n'est donc, à nouveau, pas assez robuste aux variabilités rencontrées par la suite.

A l'inverse, d'autres méthodes ont été proposées où des algorithmes sont entraînés sur des grandes quantités de données, couvrant un grand nombre de variabilités, mais ils offrent alors des performances plus faibles, insuffisantes pour l'application aéronautique et les contraintes de sécurité fortes qui s'appliquent dans ce domaine.

De manière générale, il ressort des méthodes conventionnelles et de la bibliographie la nécessité de faire un choix entre les performances de la méthode et sa couverture, c'est à dire la quantité et la variabilité des sujets sur lesquels l'algorithme de la méthode a été entrainée.

Enfin, il est à noter qu'il est nécessaire que la méthode puisse être mise en oeuvre dans un aéronef et donc avec des moyens de calcul embarqués limités, tout en déterminant l'état neurophysiologique de l'opérateur en quasi « temps réel ». Par exemple, détecter la perte de connaissance d'un pilote, en phase de décollage, doit pouvoir s'effectuer en moins de quelques secondes, typiquement jusqu'à moins de deux secondes.

Un but de l'invention est alors de proposer un dispositif électronique de surveillance d'un état neurophysiologique d'un opérateur dans un aéronef permettant de surmonter les difficultés expliquées ci-dessus, en particulier en offrant une surveillance précise, fiable et robuste aux variabilités de l'opérateur surveillé.

A cet effet, l'invention a pour objet un dispositif électronique de surveillance d'un état neurophysiologique d'un opérateur dans un aéronef, le dispositif de surveillance comprenant :
- un module de réception configuré pour recevoir une donnée d'au moins un capteur embarqué dans l'aéronef, chaque capteur étant configuré pour mesurer au moins une information relative à l'opérateur ;
- un module de catégorisation configuré pour associer à l'opérateur une catégorie parmi une liste de catégories prédéterminées à partir de la ou des données reçues ;
- un module de traitement configuré pour extraire de chaque donnée au moins un paramètre représentatif de l'état neurophysiologique de l'opérateur ;
- un module de détection configuré pour recevoir la catégorie associée à l'opérateur et le ou les paramètres représentatifs, le module de détection étant en outre configuré pour appliquer un modèle issu d'une méthode d'apprentissage automatique pour déterminer en fonction des paramètres représentatifs si l'opérateur est dans un état neurophysiologique nominal ou dans un état neurophysiologique altéré, le modèle étant choisi parmi une liste de modèles prédéterminés en fonction de la catégorie associée à l'opérateur.

Ainsi, la présente invention permet de répondre au problème posé du compromis entre performance et couverture de la surveillance. En effet, la catégorisation préalable de l'opérateur permet, malgré les combinaisons de variabilités possibles, d'assurer une couverture suffisante de la surveillance, tout en offrant des performances sur chaque modèle spécifique plus importantes. Au total, en moyennant les performances des modèles spécifiques à chaque catégorie d'opérateur, on obtient une performance de détection plus élevée qu'avec un modèle unique qui chercherait à couvrir toutes les variabilités des opérateurs. En effet, la surveillance de l'opérateur est réalisée par un modèle spécifique pour chaque catégorie, tout en assurant la couverture de toutes les combinaisons par la séparation en différentes catégories.

Suivant d'autres aspects avantageux de l'invention, le dispositif électronique de surveillance comprend une ou plusieurs des caractéristiques suivantes, prises isolément ou suivant toutes les combinaisons techniquement possibles :
- le dispositif de surveillance comprend en outre un module d'alerte configuré pour émettre un signal d'alerte lorsque le module de détection détermine que l'opérateur est dans un état neurophysiologique altéré ;
- chaque capteur est choisi parmi le groupe constitué de :
   - un capteur cardiaque, notamment un électrocardiographe ;
   - un oxymètre de pouls, notamment un capteur à photopléthysmographie ;
   - un capteur de respiration ;
   - un accéléromètre ;
   - une électrode crânienne, par exemple un électroencéphalographe ;
   - un capteur de pression agencé dans un siège de l'opérateur ;
   - un capteur de pression agencé dans un dispositif de commande propre à être actionné par l'opérateur ;
   - un capteur de sudation de l'opérateur ;
   - un capteur de réponse électrodermale ;
   - une caméra configurée pour capter au moins une image comprenant au moins une partie de l'opérateur ;
   - un microphone ;
   - un capteur infrarouge de température de peau de l'opérateur ;
   - un capteur de température interne de l'opérateur;
   - un bandeau à spectroscopie proche infrarouge.
- le module de catégorisation est configuré pour associé une catégorie en fonction au moins d'un attribut dit individuel choisi parmi le groupe constitué de : genre, âge, origine ethnique, pilosité, longueur de cheveux, présence d'éléments sur la peau du visage ;
- le module de catégorisation est configuré pour associé une catégorie en fonction au moins d'un attribut dit d'accessoire porté choisi parmi le groupe constitué de: port de lunettes, lunettes polarisés ou non, lentilles, masque chirurgical, masque à gaz, casque audio, casquette ;
- le module de traitement est configuré pour extraire de chaque donnée au moins un paramètre représentatif de l'état neurophysiologique de l'opérateur en fonction de la catégorie associée à l'opérateur ;
- le module de traitement est configuré pour extraire de chaque donnée au moins un paramètre représentatif de l'état neurophysiologique de l'opérateur en mettant en oeuvre pour chaque donnée un algorithme choisi parmi le groupe constitué de :
   - une extraction d'une caractéristique prédéterminée de la donnée associée suivi d'une méthode d'apprentissage automatique ;
   - une méthode d'apprentissage profond appliquée directement sur la donnée associée ;
   - une modélisation prédéterminée appliquée à la donnée associée.
- l'algorithme mis en oeuvre est choisi en fonction de la catégorie de l'opérateur.

L'invention concerne également un procédé de surveillance d'un état neurophysiologique d'un opérateur dans un poste de commande d'un aéronef, le procédé comprenant au moins les étapes suivantes :
- réception d'une donnée d'au moins un capteur embarqué dans l'aéronef, chaque capteur étant configuré pour mesurer au moins une information relative à l'opérateur ;
- association à l'opérateur une catégorie parmi une liste de catégories prédéterminées à partir de la ou des données reçues ;
- extraction de chaque donnée au moins un paramètre représentatif de l'état neurophysiologique de l'opérateur ;
- réception de la catégorie associée à l'opérateur et du ou des paramètres représentatifs et application d'un modèle issue d'une méthode d'apprentissage automatique pour déterminer en fonction des paramètres représentatifs si l'opérateur est dans un état neurophysiologique nominal ou dans un état neurophysiologique altéré, le modèle étant choisi parmi une liste de modèles prédéterminés en fonction de la catégorie associée à l'opérateur.

L'invention a également pour objet un programme d'ordinateur comportant des instructions logicielles qui, lorsqu'elles sont exécutées par un ordinateur, mettent en oeuvre un procédé de surveillance tel que défini ci-dessus.

Ces caractéristiques et avantages de l'invention apparaîtront plus clairement à la lecture de la description qui va suivre, donnée uniquement à titre d'exemple non limitatif, et faite en référence aux dessins annexés, sur lesquels :
**[****Fig. 1****]** la figure 1 est une représentation schématique d'un aéronef comprenant un dispositif de surveillance selon l'invention ;
**[****Fig. 2****]** la figure 2 est une représentation schématique d'un poste de commande dans l'aéronef de la figure 1,
**[****Fig. 3****]** la figure 3 est un organigramme d'un procédé de la construction et d'entrainement d'un modèle, et
**[****Fig. 4****]** la figure 4 est un organigramme d'un procédé de surveillance selon l'invention mis en oeuvre par le dispositif électronique.

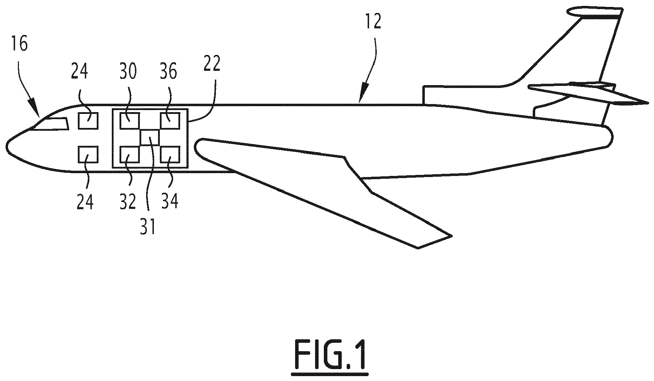

Un aéronef 12 est représenté sur la figure 1.

L'aéronef 12 est typiquement un avion, un hélicoptère, ou encore un drone. Autrement dit, l'aéronef 12 est un engin volant pilotable par un opérateur 14 via un poste de commande 16. Le poste de commande 16 est disposé à l'intérieur de l'aéronef 12 ou bien à distance de l'aéronef 12, notamment dans le cas d'un drone.

L'opérateur 14 est ici un pilote mais l'invention s'applique de manière similaire à tout opérateur de l'aéronef 12 tel qu'un co-pilote ou un opérateur radar.

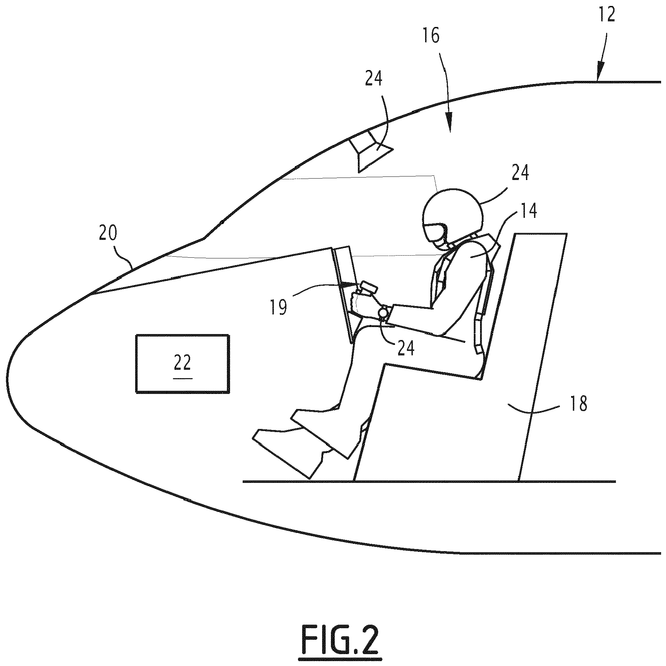

Comme visible sur la figure 2, le poste de commande 16 est ici un cockpit de l'aéronef 12. Comme visible sur la figure 1, le poste de commande 16 comporte au moins un siège 18 pour l'opérateur 14, un dispositif de commande 19 propre à être actionné par l'opérateur 14, un pare-brise 20 au moins partiellement transparent et séparant l'intérieur du cockpit de l'environnement extérieur de l'aéronef 12, au moins un capteur 24 et un dispositif électronique de surveillance 22 d'un état neurophysiologique de l'opérateur 14.

Chaque capteur 24 est configuré pour mesurer au moins une information relative à l'opérateur 14 et notamment à son état neurophysiologique, comme cela sera expliqué plus en détail par la suite.

Chaque capteur 24 est notamment un capteur dit porté ou un capteur dit déporté.

Un capteur porté est un capteur propre à être au contact physique de l'opérateur 14. L'homme du métier comprendra qu'on entend par « au contact » que le capteur 24 touche une partie de l'opérateur, avec éventuellement un habit entre le capteur et la peau de l'opérateur 14. Ainsi, le capteur porté 24 se présente par exemple sous la forme d'une montre au poignet de l'opérateur, d'un casque sur la tête de l'opérateur 14 ou d'un capteur intégré dans le dispositif de commande 19 ou dans le siège 18.

Un capteur déporté est un capteur arrangé à distance de l'opérateur 14 lors des conditions opérationnelles de vol. L'homme du métier comprendra qu'on entend par « à distance », qu'il existe un espace vide entre le capteur 24 et l'opérateur 14.

En particulier, chaque capteur 24 est choisi parmi le groupe constitué de :
- un capteur cardiaque, notamment un électrocardiographe ;
- un oxymètre de pouls, notamment un capteur à photopléthysmographie ;
- un capteur de respiration ;
- un accéléromètre ;
- une électrode crânienne, par exemple un électroencéphalographe ;
- un capteur de pression agencé dans le siège 18 de l'opérateur 14 ;
- un capteur de pression agencé dans un dispositif de commande 19 propre à être actionné par l'opérateur 14 ;
- un capteur de sudation de l'opérateur ;
- un capteur de réponse électrodermale ;
- une caméra configurée pour capter au moins une image comprenant au moins une partie de l'opérateur, notamment les yeux pour de l'oculométrie (ou « eye tracking » en anglais) ;
- un microphone ;
- un capteur infrarouge de température de peau de l'opérateur 14 ;
- un capteur de température interne de l'opérateur 14;
- un bandeau à spectroscopie proche infrarouge (aussi appelé bandeau « fNIRS ») utilisant la lumière proche infrarouge pour surveiller l'activité du cerveau.

Le dispositif électronique de surveillance 22 est configuré pour surveiller un état neurophysiologique de l'opérateur 14. L'état neurophysiologique est relatif au système nerveux de l'opérateur 14. Il est représentatif de la capacité de l'opérateur 14 à agir pour réaliser les tâches nécessaires à la sécurité de l'aéronef 12, par exemple piloter l'aéronef 12 ou répondre aux communications extérieures pour un pilote.

En particulier, l'état neurophysiologique peut être un état neurophysiologique dit « nominal », correspondant à l'état neurophysiologique attendu de l'opérateur 14 pendant un vol d'un aéronef 12, c'est-à-dire un état éveillé et lucide.

L'état neurophysiologique peut être un état neurophysiologique dit « altéré », correspondant à un état neurophysiologique dans lequel l'état neurophysiologique de l'opérateur 14 impacte sa capacité à agir pour garantir la sécurité de l'aéronef 12. Un état neurophysiologique altéré est par exemple un état de stress, de fatigue, de charge mentale trop faible ou trop élevée, d'hypo ou d'hyper-vigilance (ou tunnelisation), etc. Un état neurophysiologique altéré peut également être un état de perte, au moins partielle, de conscience du monde extérieur par l'opérateur 14, comme par exemple un état de somnolence, de sommeil ou d'évanouissement. Dans cet état neurophysiologique, l'opérateur 14 a une connaissance altérée voire inexistante de son environnement et ne peut réagir en fonction. Cet état neurophysiologique altéré est problématique lors du vol de l'aéronef 12 car l'opérateur 14 n'est pas apte à réaliser les tâches qu'il a à accomplir de manière réactive et pertinente.

A cet effet, le dispositif de surveillance 22 est configuré pour déterminer si l'opérateur 14 est dans un état neurophysiologique nominal ou dans un état neurophysiologique altéré.

En particulier, le dispositif de surveillance 22 comprend un module de réception 30, un module de catégorisation 31, un module de traitement 32 et un module de détection 34.

Le dispositif de surveillance 22 comprend avantageusement en outre un module d'alerte 36.

Le module de réception 30 est configuré pour recevoir une donnée d'au moins un capteur 24 embarqué dans l'aéronef 12, en particulier dans le poste de commande 16.

Le module de catégorisation 31 est configuré pour associer à l'opérateur 14 une catégorie parmi une liste de catégories prédéterminées à partir de la ou des données reçues par le module de réception 30.

Une catégorie est une classe à l'intérieur desquelles les éléments sont rangés suivant un certain nombre de critères. Dans le cas présent, les catégories prédéterminées permettent de classer les différents opérateurs 14 en fonction d'une liste d'attributs prédéterminés. Ainsi, une catégorie permet de caractériser les variabilités de chaque opérateur 14 en lui associant une catégorie.

La liste des catégories est prédéterminée avant les phases opérationnelles de vol, notamment à l'aide d'experts du domaine.

Le module de catégorisation 31 est configuré pour associer à l'opérateur 14 une catégorie en fonction d'attributs relatifs à l'opérateur 14.

En particulier, le module de catégorisation 31 est configuré pour associer une catégorie en fonction au moins d'un attribut dit individuel. Un attribut individuel est un attribut physique ou physiologique propre à l'opérateur 14. Chaque attribut individuel est notamment choisi parmi le groupe constitué de : genre, âge, origine ethnique, pilosité, longueur de cheveux, présence d'éléments sur la peau du visage, tels que des tatouages, du maquillage, des cicatrices, etc.

En variante ou en complément, le module de catégorisation 31 est configuré pour associé une catégorie en fonction au moins d'un attribut dit d'accessoire porté. Un attribut d'accessoire porté est un attribut relatif à un vêtement ou un objet porté par l'opérateur 14. Chaque attribut d'accessoire porté est notamment choisi parmi le groupe constitué de: port de lunettes, lunettes polarisés ou non, lentilles, masque chirurgical, masque à gaz, casque audio, casquette.

Le module de traitement 32 est configuré pour extraire de chaque donnée au moins un paramètre représentatif de l'état neurophysiologique de l'opérateur 14.

L'homme du métier comprendra que le module de catégorisation 31 et le module de traitement 32 n'utilisent pas nécessairement les mêmes données issues des capteurs. Par exemple, un capteur comme une caméra est utilisé par le module de catégorisation 31 et un autre capteur comme un capteur cardiaque est utilisé par le module de traitement 32. Toutefois, dans un mode de réalisation possible, les deux modules 31, 32 utilisent les mêmes données, par exemple les images issues d'une caméra dans le poste de commande 16.

Un paramètre représentatif de l'état neurophysiologique est un paramètre défini par exemple par des experts du domaine et permettant d'avoir une information sur l'état neurophysiologique de l'opérateur. Par exemple, un rythme de fréquence cardiaque bas, des yeux fermés sur une longue période de temps, une pression constante exercée, une position de la tête inclinée etc, sont des paramètres permettant de déterminer que l'opérateur est dans un état neurophysiologique altéré.

Avantageusement, le module de traitement 32 est configuré pour extraire de chaque donnée au moins un paramètre représentatif de l'état neurophysiologique de l'opérateur 14 en fonction de la catégorie associée à l'opérateur 14.

En particulier, le module de traitement 32 est configuré pour adapter quel paramètre parmi une liste de paramètre possible est extrait en fonction de la catégorie de l'opérateur 14.

A titre d'exemple, le module de traitement 32 peut adapter le paramètre extrait d'images en fonction du port ou non de lunettes par l'opérateur 14. Par exemple, si l'opérateur 14 ne porte pas de lunettes, le module de traitement 32 extrait des images les clignements des yeux alors que si l'opérateur 14 ne porte pas de lunettes, le module de traitement 32 extrait un mouvement de la tête.

En variante ou en complément, le module de traitement 32 est configuré pour ajuster la manière d'extraire un paramètre en fonction de la catégorie associée à l'opérateur 14.

A titre d'exemple, le seuil de détection d'un clignement des yeux de l'opérateur 14 est ajusté en fonction de la catégorie de l'opérateur 14. En effet, le seuil de détection d'un clignement d'oeil n'est pas le même entre une personne asiatique et une personne caucasienne, du fait de l'ouverture moyenne de la paupière ou des caractéristiques du pli épicanthique.

Avantageusement, le module de traitement 32 est configuré pour extraire de chaque donnée au moins un paramètre représentatif de l'état neurophysiologique de l'opérateur 14 en effectuant une extraction d'une caractéristique prédéterminée de la donnée associée suivi d'une méthode d'apprentissage automatique.

A titre d'exemple, la caractéristique est la position de la tête de l'opérateur 14 extraite d'une vidéo obtenue par une caméra. Puis une méthode d'apprentissage automatique est mise en oeuvre pour traiter la position de la tête au cours du temps et en déduire le paramètre représentatif de l'état neurophysiologique de l'opérateur 14.

Une méthode d'apprentissage automatique (ou « Machine Learning » en anglais) permet d'obtenir un modèle capable de résoudre des tâches sans être explicitement programmé pour chacune de ces tâches. L'apprentissage automatique comporte deux phases. La première phase consiste à définir un modèle à partir de données présentes dans une base de données d'apprentissage, appelées aussi observations. La définition du modèle consiste notamment ici à l'entraîner à reconnaître un état neurophysiologique délétère. Cette phase dite d'apprentissage est généralement réalisée préalablement à l'utilisation pratique du modèle. La seconde phase correspond à l'utilisation du modèle : le modèle étant défini, de nouvelles données peuvent alors être soumises au modèle afin de déterminer l'état neurophysiologique de l'opérateur 14.

En variante, le module de traitement 32 est configuré pour extraire de chaque donnée au moins un paramètre représentatif de l'état neurophysiologique de l'opérateur 14 en mettant en oeuvre une méthode d'apprentissage profond appliquée directement sur la donnée associée.

Une méthode d'apprentissage profond (ou « Deep Learning ») est une technique reposant sur le modèle des réseaux neuronaux, ou réseaux de neurones : des dizaines voire des centaines de couches de neurones sont empilées pour apporter une plus grande complexité au modèle. En particulier, un réseau de neurones est en général composé d'une succession de couches dont chacune prend ses entrées sur les sorties de la précédente. Chaque couche est composée d'une pluralité de neurones, prenant leurs entrées sur les neurones de la couche précédente. À chaque synapse entre neurones est associée un poids synaptique, de sorte que les entrées reçues par un neurone sont multipliées par ce poids, puis additionnées par ledit neurone. Le réseau neuronal est optimisé grâce aux ajustements des différents poids synaptiques pendant son entrainement en fonction des données présentes dans la base de données d'apprentissage. Le réseau de neurones ainsi optimisé est alors le modèle. Un nouveau jeu de donnés peut alors être donné en entrée du réseau de neurones qui fournit alors le résultat de la tâche pour laquelle il a été entrainé.

En variante, le module de traitement 32 est configuré pour extraire de chaque donnée au moins un paramètre représentatif de l'état neurophysiologique de l'opérateur 14 en mettant en oeuvre une modélisation prédéterminée appliquée à la donnée associée.

La modélisation prédéterminée est par un exemple un modèle physique comprenant un ensemble de règles prédéterminées par un expert du domaine.

Dans un mode de réalisation avantageux, le module de traitement 32 est configuré pour extraire de chaque donnée au moins un paramètre représentatif de l'état neurophysiologique de l'opérateur 14 en mettant en oeuvre un algorithme choisi en fonction de la catégorie de l'opérateur 14 parmi le groupe constitué de:
- une extraction d'une caractéristique prédéterminée de la donnée associée suivi d'une méthode d'apprentissage automatique ;
- une méthode d'apprentissage profond appliquée directement sur la donnée associée ;
- une modélisation prédéterminée appliquée à la donnée associée.

A titre d'exemple, pour une catégorie dans lequel l'entrainement de la méthode d'apprentissage profond associé a été fait sur un grand nombre de données et avec des bons résultats de performance, cette méthode sera privilégiée. A l'inverse, pour une catégorie avec peu de données d'expérience et où les méthodes d'apprentissage automatique sont moins performantes, une modélisation prédéterminée est privilégiée pour éviter les faux positifs.

Le module de détection 34 est configuré pour recevoir la catégorie associée à l'opérateur 14 déterminée par le module de catégorisation 31 et le ou les paramètres représentatifs déterminé(s) par le module de traitement 32.

Le module de détection 34 est en outre configuré pour appliquer un modèle issu d'une méthode d'apprentissage automatique pour déterminer en fonction des paramètres représentatifs si l'opérateur 14 est dans un état neurophysiologique nominal ou dans un état neurophysiologique altéré.

La méthode d'apprentissage automatique utilisée par le module de détection 34 est différente, ou éventuellement similaire à celle utilisée par le module de traitement 32.

Le modèle est choisi parmi une liste de modèles prédéterminés en fonction de la catégorie associée à l'opérateur 14.

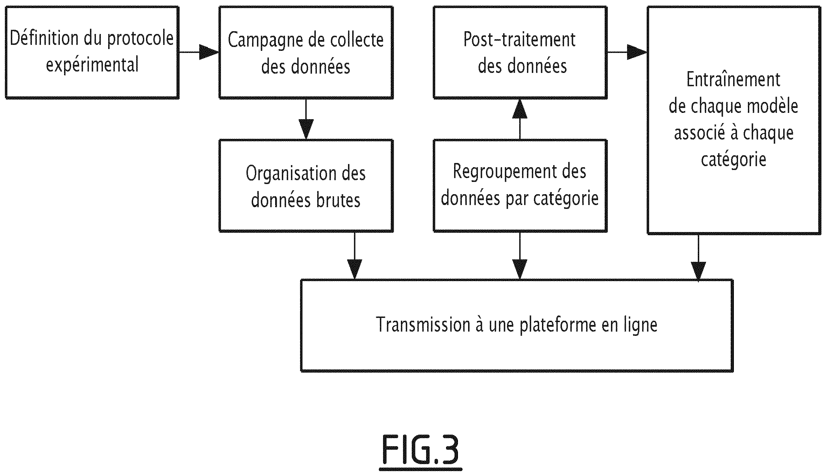

La figure 3 représente un organigramme du procédé de la construction et de l'entrainement de tels modèles prédéterminés.

Dans un premier temps, le protocole expérimental est mis en place. Il s'agit de définir pour l'état neurophysiologique que l'on veut surveiller :
- la manière de générer cet état chez des sujets,
- les capteurs pertinents pour le surveiller,
- les moyens de collecter la « vérité terrain », aussi appelée le label, i.e. l'état dans lequel se trouve le sujet (par exemple : fatigue normale ou fatigue extrême),
- le nombre de sujets nécessaire afin d'assurer une représentativité suffisante du modèle, ainsi que les variabilités interindividuelles ou en termes d'objets portés que l'on veut adresser,
- l'environnement de collecte (laboratoire, vol, etc.) et sa représentativité par rapport à l'utilisation finale du système,
- les biais potentiels liés à l'expérimentation.

Durant cette phase de définition du protocole, le procédé comprend une étape d'identification des différentes combinaisons de variabilités devant être surveillées. En particulier, il est étudié pendant cette étape quels paramètres sont significatifs et pertinents pour caractériser l'état neurophysiologique de l'opérateur 14 en fonction de chaque catégorie, notamment avec l'aide d'experts du domaine.

Les combinaisons de variabilités peuvent ne dépendre que d'un paramètre, comme par exemple faire un modèle pour les hommes et un pour les femmes, mais il peut aussi en contenir un grand nombre, par exemple un modèle pour les hommes asiatiques portant des lunettes. De ce nombre de combinaisons dépend le nombre de modèles qu'il faut créer.

Suite à cela, les données sont collectées durant les campagnes. Il en résulte une base de données physiologiques labellisées, i.e. associées à un état physiologique précis.

Ces données étant brutes, elles sont traitées. La qualité des données est alors estimée en fonction de critères complémentaires. Par exemple, on peut déterminer la qualité de la détection d'un visage grâce à un algorithme de « facetracking ». On vérifie également l'échantillonnage des données, la présence d'artefacts ou de bruit capteur. On peut également enlever la tendance des signaux, i.e. des éléments basse fréquence non pertinents pour le monitoring.

A l'issue des collectes des données, les données sont regroupées dans les combinaisons identifiées d'attributs afin de former les différentes combinaisons. Un modèle est alors développé pour chaque catégorie, *i.e.* chaque combinaison.

Ainsi, après avoir récolté les données d'entrainement sur une pluralité d'opérateurs, ces différents opérateurs sont par exemple classés en fonction de la variabilité « genre », *i.e.* homme ou femme, ou par exemple encore en fonction de la variabilité « origine ethnique », *i.e.* caucasien, africain, asiatique, méditerranéen, etc. Les catégories peuvent également être formées en effectuant des combinaisons de deux variabilités ou plus pour chaque opérateur, par exemple homme et asiatique ou femme et australienne, et le modèle entraîné sur les données issues uniquement des hommes asiatiques ou des femmes australiennes dans cet exemple.

A chaque étape du procédé, les éléments sont avantageusement transmis à une plateforme en ligne habilitée à héberger des données GPDR (Règlement général sur la protection des données) et HDS (hébergement de données de santé).

Le module d'alerte 36 est configuré pour émettre un signal d'alerte lorsque le module de détection 34 détermine que l'opérateur est dans un état neurophysiologique altéré.

Le signal d'alerte est par exemple un signal sonore émis dans le poste de commande 16 dans le but de faire revenir l'opérateur 14 à un état neurophysiologique nominal.

En variante ou en complément, le signal d'alerte est par exemple un signal envoyé à un système de contrôle de l'aéronef 12 pour passer en mode automatique et qu'ainsi les taches à réaliser par l'opérateur 14 soient réalisées de manière autonome sans l'intervention de l'opérateur 14. Notamment, lorsque l'opérateur 14 est un pilote, l'aéronef 12 passe en pilote automatique.

En variante ou en complément, le signal d'alerte est par exemple un signal de communication vers un dispositif de contrôle externe à l'aéronef 12 tel qu'une tour de contrôle.

Dans l'exemple de la figure 1, le dispositif électronique de surveillance 22 comprend une unité de traitement d'informations formée par exemple d'une mémoire et d'un processeur associé à la mémoire. Le module de réception 30, le module de catégorisation 31, le module de traitement 32, le module de détection 34, et en complément facultatif, le module d'alerte 36 sont réalisés chacun sous forme d'un logiciel, ou d'une brique logicielle, exécutables par le processeur. La mémoire est alors apte à stocker un logiciel de réception, un logiciel de catégorisation, un logiciel de traitement, un logiciel de détection et en complément facultatif, un logiciel d'alerte. Le processeur est alors apte à exécuter chacun de ces logiciels.

En variante non représentée, le module de réception 30, le module de catégorisation 31, le module de traitement 32, le module de détection 34, et en complément facultatif, le module d'alerte 36 sont réalisés chacun sous forme d'un composant logique programmable, tel qu'un FPGA (de l'anglais Field Programmable Gate Array), ou encore sous forme d'un circuit intégré dédié, tel qu'un ASIC (de l'anglais Application Spécifie Integrated Circuit).

Lorsque le dispositif électronique 22 est réalisé sous forme d'un ou plusieurs logiciels, c'est-à-dire sous forme d'un programme d'ordinateur, il est en outre apte à être enregistré sur un support, non représenté, lisible par ordinateur. Le support lisible par ordinateur est par exemple, un médium apte à mémoriser les instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple EPROM, EEPROM, FLASH, NVRAM), une carte magnétique ou une carte optique. Sur le support lisible est alors mémorisé un programme d'ordinateur comportant des instructions logicielles.

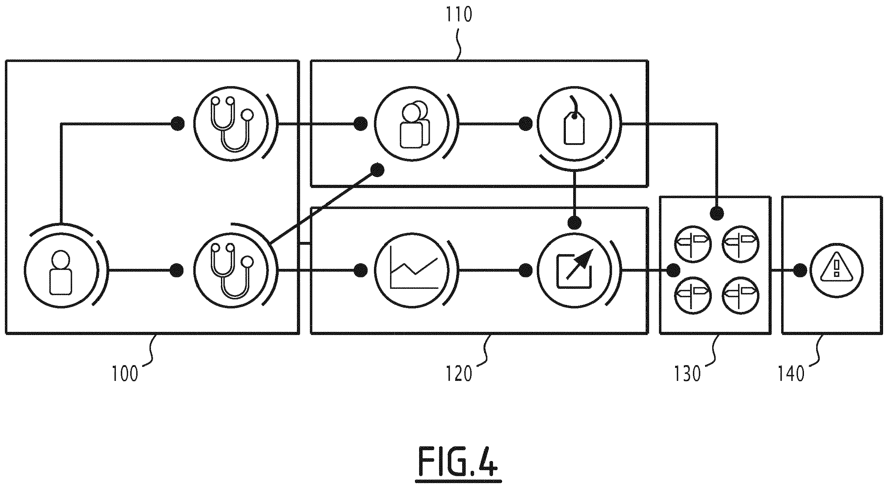

Le fonctionnement du dispositif électronique de surveillance 22 selon l'invention va désormais être expliqué à l'aide de la figure 4 représentant un organigramme du procédé, selon l'invention, de surveillance d'un état neurophysiologique d'un opérateur 14 dans un poste de commande 16 d'un aéronef 12.

Initialement, l'aéronef 12 est en situation opérationnelle de vol, volant par exemple en destination d'un aéroport.

Au moins un opérateur 14 est présent dans le poste de commande 16 de l'aéronef 12. L'opérateur 14 est par exemple un pilote, comme illustré ici.

A titre d'exemple, le pilote est par exemple un homme asiatique portant des lunettes.

Le procédé comprend une étape initiale 100 de réception par le module de réception 30 d'une donnée d'au moins un capteur 24 embarqué dans l'aéronef 12.

Chaque capteur 24 est configuré pour mesurer au moins une information relative au pilote.

Ici, comme illustré par la figure 2, le module de réception 30 reçoit par exemple les images du visage du pilote issues d'une caméra disposée dans le poste de commande 16 et les données cardiaques du pilote à partir de la montre au poignet du pilote.

Puis, le procédé comprend une étape 110 d'association à l'opérateur 14 d'une catégorie parmi une liste de catégories prédéterminées à partir de la ou des données reçues par le module de catégorisation 31.

Dans l'exemple précédent, le module de catégorisation 31 détermine à partir des images que le genre du pilote est « homme », que l' « origine ethnique » du pilote est « asiatique » et que l'objet porté sur le visage est « lunettes ». A partir de ces attributs, le module de catégorisation 31 associe le pilote à la catégorie « homme asiatique à lunettes » qui fait partie des catégories prédéterminées à disposition du module de catégorisation 31.

Puis, le procédé comprend une étape 120 d'extraction de chaque donnée d'au moins un paramètre représentatif de l'état neurophysiologique de l'opérateur 14.

Dans l'exemple précédent, le module de traitement 32 extrait des données cardiaques du pilote notamment la fréquence cardiaque et la compare à une valeur seuil de repos en dessous de laquelle il y a une suspicion d'état neurophysiologique altéré du pilote. En variante ou en complément le module de traitement 32 extrait des images le mouvement de la tête du pilote.

Avantageusement, le module de traitement 32 extrait de chaque donnée au moins un paramètre représentatif de l'état neurophysiologique de l'opérateur en fonction de la catégorie associée à l'opérateur 14.

Dans l'exemple précédent, le module de traitement 32 évite d'extraire la fréquence de clignements de ses yeux car cette extraction est moins fiable de par la présence des lunettes.

Puis, le procédé comprend une étape 130 de réception de la catégorie associée à l'opérateur 14 et du ou des paramètres représentatifs par le module de détection 34 et application d'un modèle issue d'une méthode d'apprentissage automatique pour déterminer en fonction des paramètres représentatifs si l'opérateur 14 est dans un état neurophysiologique nominal ou dans un état neurophysiologique altéré. Le modèle est choisi parmi une liste de modèles prédéterminés en fonction de la catégorie associée à l'opérateur 14.

Toujours dans l'exemple précédent, le module de détection 34 met en oeuvre le modèle associé à la catégorie « homme asiatique à lunettes ». Ce modèle a été entrainé spécialement sur des données d'entrainement relatives à cette catégorie de pilote et est donc particulièrement performant pour déterminer l'état neurophysiologique de ce pilote. Ici, à partir de la fréquence cardiaque et/ou des mouvements de la tête du pilote, le module de détection 34 détermine si le pilote est dans un état neurophysiologique nominal ou dans un état altéré. Par exemple, si le module de détection 34 reçoit une fréquence cardiaque basse et/ou un balancement de la tête latéralement, le modèle permet de déterminer éventuellement un état neurophysiologique altéré du pilote.

Avantageusement, le procédé comprend une étape 140 d'émission d'un signal d'alerte lorsque le module de détection 34 détermine que l'opérateur 14 est dans un état neurophysiologique altéré.

On conçoit alors que la présente invention présente un certain nombre d'avantages.

En effet, le dispositif selon l'invention permet une surveillance de l'état neurophysiologique de l'opérateur 14 performante tout en étant robuste aux variabilités des opérateurs surveillés. La catégorisation préalable de l'opérateur 14 permet d'assurer une couverture suffisante de la surveillance, tout en offrant des performances sur chaque modèle spécifique plus importantes. En effet, la surveillance de l'opérateur 14 est réalisée par un modèle associé à chaque catégorie, entrainé spécifiquement sur des données relatives à cette catégorie. Chaque modèle spécifique offre donc une performance plus élevée qu'avec un modèle unique qui chercherait à couvrir toutes les variabilités possibles des opérateurs.

L'invention permet donc une meilleure détection des états neurophysiologiques altérés des opérateurs et permet de réduire les faux positifs. Ainsi l'invention permet d'améliorer la sécurité de l'aéronef 12.

## Revendications

1. Dispositif électronique de surveillance (22) d'un état neurophysiologique d'un opérateur (14) dans un poste de commande (16) d'un aéronef (12), le dispositif de surveillance (22) comprenant :
- un module de réception (30) configuré pour recevoir une donnée d'au moins un capteur (24) embarqué dans l'aéronef (12), chaque capteur étant configuré pour mesurer au moins une information relative à l'opérateur (14) ;
- un module de catégorisation (31) configuré pour associer à l'opérateur (14) une catégorie parmi une liste de catégories prédéterminées à partir de la ou des données reçues ;
- un module de traitement (32) configuré pour extraire de chaque donnée au moins un paramètre représentatif de l'état neurophysiologique de l'opérateur (14) ;
- un module de détection (34) configuré pour recevoir la catégorie associée à l'opérateur (14) et le ou les paramètres représentatifs, le module de détection (30) étant en outre configuré pour appliquer un modèle issu d'une méthode d'apprentissage automatique pour déterminer en fonction des paramètres représentatifs si l'opérateur (14) est dans un état neurophysiologique nominal ou dans un état neurophysiologique altéré, le modèle étant choisi parmi une liste de modèles prédéterminés en fonction de la catégorie associée à l'opérateur (14).

2. Dispositif de surveillance (22) selon la revendication 1, dans lequel le dispositif de surveillance (22) comprend en outre un module d'alerte (36) configuré pour émettre un signal d'alerte lorsque le module de détection (34) détermine que l'opérateur (14) est dans un état neurophysiologique altéré.

3. Dispositif de surveillance (22) selon la revendication 1 ou 2, dans lequel chaque capteur (24) est choisi parmi le groupe constitué de :
- un capteur cardiaque, notamment un électrocardiographe ;
- un oxymètre de pouls, notamment un capteur à photopléthysmographie ;
- un capteur de respiration ;
- un accéléromètre ;
- une électrode crânienne, par exemple un électroencéphalographe ;
- un capteur de pression agencé dans un siège (18) de l'opérateur (14) ;
- un capteur de pression agencé dans un dispositif de commande (19) propre à être actionné par l'opérateur (14) ;
- un capteur de sudation de l'opérateur ;
- un capteur de réponse électrodermale ;
- une caméra configurée pour capter au moins une image comprenant au moins une partie de l'opérateur (14) ;
- un microphone ;
- un capteur infrarouge de température de peau de l'opérateur (14) ;
- un capteur de température interne de l'opérateur (14) ;
- un bandeau à spectroscopie proche infrarouge.

4. Dispositif de surveillance (22) selon l'une quelconque des revendications précédentes, dans lequel le module de catégorisation (31) est configuré pour associer une catégorie en fonction au moins d'un attribut dit individuel choisi parmi le groupe constitué de : genre, âge, origine ethnique, pilosité, longueur de cheveux, présence d'éléments sur la peau du visage.

5. Dispositif de surveillance (22) selon l'une quelconque des revendications précédentes, dans lequel le module de catégorisation (31) est configuré pour associer une catégorie en fonction au moins d'un attribut dit d'accessoire porté choisi parmi le groupe constitué de: port de lunettes, lunettes polarisés ou non, lentilles, masque chirurgical, masque à gaz, casque audio, casquette.

6. Dispositif de surveillance (22) selon l'une quelconque des revendications précédentes, dans lequel le module de traitement (32) est configuré pour extraire de chaque donnée au moins un paramètre représentatif de l'état neurophysiologique de l'opérateur en fonction de la catégorie associée à l'opérateur (14).

7. Dispositif de surveillance (22) selon l'une quelconque des revendications précédentes, dans lequel le module de traitement (32) est configuré pour extraire de chaque donnée au moins un paramètre représentatif de l'état neurophysiologique de l'opérateur (14) en mettant en oeuvre pour chaque donnée un algorithme choisi parmi le groupe constitué de :
- une extraction d'une caractéristique prédéterminée de la donnée associée suivi d'une méthode d'apprentissage automatique ;
- une méthode d'apprentissage profond appliquée directement sur la donnée associée ;
- une modélisation prédéterminée appliquée à la donnée associée.

8. Dispositif de surveillance (22) selon la revendication 7, dans lequel l'algorithme mis en oeuvre est choisi en fonction de la catégorie de l'opérateur (14).

9. Procédé de surveillance d'un état neurophysiologique d'un opérateur (14) dans un poste de commande (16) d'un aéronef (12), le procédé de surveillance comprenant au moins les étapes suivantes :
- réception (100) d'une donnée d'au moins un capteur (24) embarqué dans l'aéronef (12), chaque capteur étant configuré pour mesurer au moins une information relative à l'opérateur (14) ;
- association (110) à l'opérateur (14) une catégorie parmi une liste de catégories prédéterminées à partir de la ou des données reçues ;
- extraction (120) de chaque donnée au moins un paramètre représentatif de l'état neurophysiologique de l'opérateur (14) ;
- réception (130) de la catégorie associée à l'opérateur et du ou des paramètres représentatifs et application d'un modèle issue d'une méthode d'apprentissage automatique pour déterminer en fonction des paramètres représentatifs si l'opérateur (14) est dans un état neurophysiologique nominal ou dans un état neurophysiologique altéré, le modèle étant choisi parmi une liste de modèles prédéterminés en fonction de la catégorie associée à l'opérateur (14).

10. Programme d'ordinateur comportant des instructions logicielles qui, lorsqu'elles sont exécutées par un ordinateur, mettent en oeuvre un procédé de surveillance selon la revendication précédente.
